(19) Europäisches Patentamt
European Patent Office
Office européen des brevets

(11) **EP 2 478 372 B1**

(12) **EUROPEAN PATENT SPECIFICATION**

(45) Date of publication and mention of the grant of the patent:
**09.11.2016 Bulletin 2016/45**

(51) Int Cl.:
***C40B 40/04*** *(2006.01)*　　***G01N 33/68*** *(2006.01)*

(21) Application number: **10754327.4**

(22) Date of filing: **15.09.2010**

(86) International application number:
**PCT/EP2010/063569**

(87) International publication number:
**WO 2011/032994 (24.03.2011 Gazette 2011/12)**

(54) **Improved method and array for a highly sensitive detection and quantification of biomolecules using secondary ion mass spectrometry (SIMS)**

Verbessertes Verfahren und Array zur hochempfindlichen Detektion und Quantifizierung von Biomolekülen mittels Sekundärionenmassenspektrometrie (SIMS)

Procédé amélioré et puce pour la détection hautement sensible et la quantification de biomolécules à l'aide de spectrométrie de masse ionique secondaire (SIMS)

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **15.09.2009 EP 09305855**

(43) Date of publication of application:
**25.07.2012 Bulletin 2012/30**

(73) Proprietor: **BIOSIMS TECHNOLOGIES**
**76000 Rouen (FR)**

(72) Inventors:
  • **RIPOLL, Camille**
    **F-76420 Bihorel (FR)**
  • **NORRIS, Victor**
    **F-76230 Bois Guillaume (FR)**
  • **LEGENT, Guillaume**
    **F-76300 Sotteville Les Rouen (FR)**
  • **DELAUNE, Anthony**
    **F-76270 Saint Saire (FR)**

(74) Representative: **Paris, Fabienne**
    **Ernest Gutmann - Yves Plasseraud S.A.S.**
    **3, rue Auber**
    **75009 Paris (FR)**

(56) References cited:
  • **BELU A M ET AL: "Enhanced TOF-SIMS imaging of a micropatterned protein by stable isotope protein labeling." ANAL CHEM, vol. 73, no. 2, 15 January 2001 (2001-01-15), pages 143-150, XP002547178**
  • **GALLI MARXER C ET AL: "Supported membrane composition analysis by secondary ion mass spectrometry with high lateral resolution." BIOPHYS J, vol. 88, no. 4, April 2005 (2005-04), pages 2965-2975, XP002547179**
  • **PETT-RIDGE J ET AL: "Microarrays + NanoSIMS: Linking Microbial Identity and Function with "NanoSIP"" SECOND ANNUAL DOE JOINT GENOME INSTITUTE USER MEETING, [Online] 28 March 2007 (2007-03-28), - 30 March 2007 (2007-03-30) XP002547180 Walnut Creek, California Retrieved from the Internet: URL:http://www.jgi.doe.gov/meetings/usermt g07/2007_jgi_user_meeting.pdf> [retrieved on 2009-09-23]**

- BRODIE E ET AL: "Abstract 99: Profiling Microbial Identity and Activity: Novel Applications of NanoSIMS and High Density Microarrays" GENOMICS:GTL AWARDEE WORKSHOP VI AND METABOLIC ENGINEERING WORKING GROUP INTERAGENCY CONFERENCE ON METABOLIC ENGINEERING 2008, [Online] 10 February 2008 (2008-02-10), - 13 February 2008 (2008-02-13) XP002547181 Bethesda, Maryland Retrieved from the Internet: URL:http://genomicsgtl.energy.gov/pubs/200 8abstracts/2008GTLabstracts.pdf> [retrieved on 2009-09-23]

- BRANDT O ET AL: "Development towards label- and amplification-free genotyping of genomic DNA" APPL SURFACE SCI, vol. 252, no. 19, 30 July 2006 (2006-07-30), pages 6935-6940, XP024892918
- LECHENE C ET AL: "High-resolution quantitative imaging of mammalian and bacterial cells using stable isotope mass spectrometry" J BIOL, vol. 5, no. 6, 5 October 2006 (2006-10-05) , pages 20.1-20.30, XP021021301

**Description**

**FIELD OF THE APPLICATION OR INVENTION**

The application describes and the invention relates to a method for detection and quantification of biomolecules, such as DNA, RNA or proteins, using isotope labeling and secondary ion mass spectrometry, and arrays designed for carrying out said method.

**BACKGROUND OF THE APPLICATION OR INVENTION**

In the early sixties, Castaing and Slodzian developed mass-filtered emission ion microscopy using secondary ions, which is part of a technique later named secondary ion mass spectrometry (SIMS). With this technique, a beam of ions (the primary ion beam) is used as a probe to sputter the surface atomic layers of a sample into atoms or atomic clusters, a small fraction of which are ionized. In a SIMS instrument, these secondary ions are separated according to mass and are then used to measure a secondary ion current to create, for example, a quantitative atomic mass image of the analyzed surface.

SIMS has become a major tool in semiconductor and surface science studies, geochemistry, the characterization of organic material, and cosmochemistry. However, ion microscopy has been for a long time considered only as a marginal method for solving problems in life sciences, due mainly to poor lateral resolution (1-0.5 $\mu$m) and insufficient mass separation power.

Technological and conceptual improvements led to significant progress in both lateral resolving power and mass resolution, in particular due to the use of a finely focused primary ion beam. SIMS microscopy has therefore become a very powerful imaging tool. For example, Lechene et al. were able using the SIMS technique to image individual stereocilia, the mechanosensory organelles of the inner cells of the cochlea (Lechene at al. Journal of Biology, 2006, 5:20). In another experiment, they were able to study the nitrogen fixation in bacteria cultured in a $^{15}$N atmosphere. The use of SIMS technique also allowed Lechene et al. to localize, quantify and compare nitrogen fixation in single cells and subcellular structures (Lechene et al. Science 2007, 317:1563). Thus, SIMS technology is now widely used for imaging cells or tissues, and is a powerful tool for diagnostic.

SIMS technique was also used to detect hybridization of unlabelled DNA to microarrays of peptide nucleic acids (PNA) (Brandt et al, 2003, Nucleic Acids Research, 31: 19). In these experiments, PNA/DNA or PNA/RNA duplexes were visualized by SIMS detecting the phosphates that are an integral part of the nucleic acids but are completely missing in PNA. Belu et al. 2001 (Anal. Chem. 73(2): 143-150) relates to enhanced TOF-SIMS imaging of a micropatterned protein by stable isotope protein labeling.

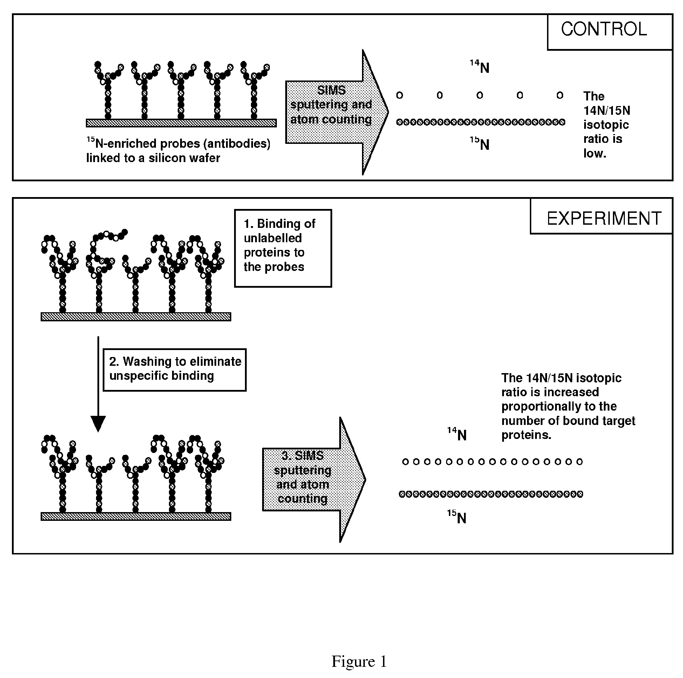

The application or invention aims to provide a method for detecting and quantifying the presence or absence of a number of biomolecules in a sample using the SIMS technique. The method described in Brandt et al. presents the following drawbacks: (i) it can only be applied with PNA probes or probes that do not contain phosphates and (ii) it does not allow quantification of the interaction probe/target. In a previous patent application, the Applicant aimed to provide a universal method that can be applied to a great number of samples for the detection and the quantification of a great number of interaction probe/targets in each sample using the SIMS technique, the detection and the quantification of said interactions being determined by the calculation of the isotopic ratio probe/target (see Figure 1). In the application or invention, the Applicant aims to provide a more accurate method. Indeed, the inventors found that coating the array with a hydrophilic polymer that does not contain the common secondary ions corresponding to the rare isotope used for labeling the probes gives an additional advantage to the method, as it prevents the non-specific adsorption of any hydrophobic molecules such as nucleic acids, peptides, or proteins present in the sample to be tested and thus minimizes the background.

**SUMMARY OF THE APPLICATION OR INVENTION**

The application describes an array comprising a substantially planar substrate having a conducting surface and a number of discrete areas containing probes being labeled with at least one rare, stable or unstable isotope or exogenous isotope, wherein the array or the discrete areas are coated with a hydrophilic polymer that does not contain the common secondary ions corresponding to the rare or exogenous isotope used for labeling the probes.

The invention relates to an array comprising a planar substrate having a conducting surface and a number of discrete areas containing molecular probes being labeled with at least one rare heavy stable isotope selected in the group of $^{15}$N, $^{33}$S, $^{34}$S and $^{36}$S, or with at least one exogenous isotope selected in the group of $^{79}$Br and $^{81}$Br, wherein the array or the discrete areas are coated with a hydrophilic polymer that does not contain the common secondary ions $^{14}$N, $^{32}$S, $^{80}$Br, respectively, corresponding to the rare or exogenous isotope used for labeling the molecular probes.

The invention also relates to an array comprising a planar substrate having a conducting surface and a number of discrete areas containing molecular probes being labeled with at least one rare, stable, isotope selected in the group $^{2}$H, $^{13}$C, $^{15}$N, $^{17}$O, $^{18}$O, $^{33}$S, $^{34}$S and $^{36}$S, or with at least one rare, unstable, isotope selected in the group $^{3}$H and $^{14}$C or with at least one exogenous isotope selected in the group $^{79}$Br and $^{81}$Br, wherein the array or the discrete areas are coated with a hydrophilic polymer that does not contain the common secondary ions $^{1}$H, $^{12}$C, $^{14}$N, $^{16}$O $^{32}$S $^{80}$Br, respectively, corresponding to the rare or exogenous isotope used for labeling the molecular probes, but that contains at least one of said rare stable isotope or exogenous isotope used for labeling the molecular probes.

According to one embodiment of the application or invention, said substantially planar substrate having a conducting surface is a silicon wafer.

According to one embodiment of the application or invention, said hydrophilic polymer that does not contain nitrogen is selected in the group comprising monosaccharide, disaccharide, polysaccharide, and synthetic hydrophilic polymer that does not contain nitrogen.

According to one embodiment of the application or invention, said synthetic hydrophilic polymer is polyethylene glycol.

According to one embodiment of the application, said probes are labeled with at least one heavy stable isotope selected in the group $^{2}$H, $^{13}$C, $^{15}$N, $^{17}$O $^{18}$O, $^{33}$S $^{34}$S and $^{36}$S, or with at least one unstable isotope selected in the group $^{3}$H and $^{14}$C or with at least one exogenous isotope selected in the group of $^{79}$Br and $^{81}$Br.

According to an embodiment of the invention, said probes are molecular probes labeled with at least one heavy stable isotope selected in the group $^{15}$N, $^{33}$S, $^{34}$S and $^{36}$S.

According to one embodiment of the application or invention, said array is a microarray wherein each discrete area has one of the dimensions length, width or diameter being from 1 $\mu$m to 1000 $\mu$m

According to one embodiment of the application, each discrete area is a microwell that comprises probes being labeled with at least one rare, stable or unstable isotope or exogenous isotope.

According to an embodiment of the invention, each discrete area is a microwell that comprises molecular probes labeled with at least one rare heavy stable isotope selected in the group of $^{15}$N , $^{33}$S, $^{34}$S and $^{36}$S, or with at least one exogenous isotope selected in the group of $^{79}$Br and $^{81}$Br.

According to one embodiment of the application or invention, said array is a nanoarray wherein each discrete area has one of the dimensions length, width or diameter being from 1 nm to 1000 nm.

According to one embodiment of the application, each discrete area is a nanowell that comprises probes being labeled with at least one rare, stable or unstable isotope or exogenous isotope.

According to an embodiment of the invention, each discrete area is a nanowell that comprises molecular probes labeled with at least one rare heavy stable isotope selected in the group of $^{15}$N, $^{33}$S, $^{34}$S and $^{36}$S, or with at least one exogenous isotope selected in the group of $^{79}$Br and $^{81}$Br.

According to one embodiment of the application, each microwell comprises a number of nanowells, and said nanowells comprise the probes being labeled with at least one rare, stable or unstable isotope or exogenous isotope.

According to an embodiment of the invention, each microwell comprises a number of nanowells, and said nanowells comprise the molecular probes labeled with at least one rare heavy stable isotope selected in the group of $^{15}$N, $^{33}$S, $^{34}$S and $^{36}$S, or with at least one exogenous isotope selected in the group of $^{79}$Br and $^{81}$Br.

The application describes a method for detecting and quantifying in at least one sample the presence or absence of at least one biomolecule, comprising:

(a) contacting said at least one sample with an array of the application as described here above,
(b) washing and drying the array,
(c) detecting and counting by SIMS the common secondary ions along with the corresponding rare secondary ions.

The invention relates to an *in vitro* method for detecting and quantifying in at least one sample the presence or absence of at least one biomolecule, comprising:

(a) contacting said at least one sample with an array of the invention,
(b) washing and drying the array,
(c) detecting and counting by SIMS the common secondary ions along with the corresponding rare secondary ions.

According to one embodiment of the application or invention, each sample to be tested is contacted with one or more discrete area of the array.

According to one embodiment of the application or invention, said sample to be tested is a single cell.

According to one embodiment of the application or invention, said method is for determining a molecular atlas of the sample tested, wherein said molecular atlas is the determination of the transcriptome, proteome, lipidome, metabolome, glycome and/or interactome of said sample.

According to one embodiment of the application or invention, said method is for predicting a predisposition to a disease, or for diagnosing a disease in a subject in need thereof.

According to one embodiment of the application or invention, said method is for monitoring the efficacy of a therapeutic agent administrated to a subject to treat a disease.

According to one embodiment of the application or invention, said method is for screening therapeutic agents.

## BRIEF DESCRIPTION OF THE DRAWINGS

**Figure 1:** Schematic illustration of the principle of SIMS detection of the binding of probes to their target proteins. The biomolecules (probes and targets) are fragmented down to the atomic level (dynamic SIMS conditions). Hence, each individual couple of probe/target gives hundreds of $^{15}N$ and $^{14}N$ atoms (in the form of $CN^-$ molecular secondary ions in a real experiment), which are easily counted in the SIMS analyser (amplification). The measurement of the increase in the $^{14}N/^{15}N$ isotopic ratio allows the quantification of those target proteins bound to antibodies.

**Figure 2:** increase or decrease in the DIR depending on whether the probe or the target is labeled.

**Figure 3:** isotopic fraction of $^{15}N$.

**Figure 4:** isotopic fraction of $^{13}C$.

**Figure 5:** SIMS analysis of antibody-target interactions. Wabo, wafer with antibody (anti-CD34) only; Wtap, wafer with antibody plus target protein (CD34); Wbsa, wafer with antibody plus control protein (Bovine Serum Albumin). Detection Isotopic Ratios $C^2C^{15}N/C^{12}C^{15}N+^{12}C^{14}N)$) were obtained with successive scans (i.e. at a different depths) in a NanoSIMS 50.

**Figure 6:** Mean values of antibody-target interactions. Wabo, wafer with antibody (anti-CD34) only; Wtap, wafer with antibody plus target protein (CD34); Wbsa, wafer with antibody plus control protein (Bovine Serum Albumin). Detection Isotopic Ratios $C^2C^{15}N/C^2C\ ^{15}N+^{12}C^{14}N)$) were obtained with a NanoSIMS 50 for scans 2-12 at four or five different places in the antibody spot on each wafer. Mean values plus the standard error on the mean are shown.

**Figure 7:** Prototype of protein chip technology compatible with SIMS developed according to Experiment 4 below.

**Figure 8:** Curve of the isotopic fraction as a function of the amount of target molecules, as performed in Experiment 4.

## DETAILED DESCRIPTION OF THE APPLICATION OR INVENTION

### Definitions

- The term "nucleic acid" as used herein means a polymer composed of nucleotides, e.g., deoxyribonucleotides or ribonucleotides, or compounds produced synthetically such as PNA which can hybridize with naturally occurring nucleic acids in a sequence specific manner analogous to that of two naturally occurring nucleic acids, e.g., can participate in Watson-Crick base-pairing interactions.
- The terms "ribonucleic acid" and "RNA" as used herein mean a polymer composed of ribonucleotides.
- The terms "deoxyribonucleic acid" and "DNA" as used herein mean a polymer composed of deoxyribonucleotides.
- The term "oligonucleotide" as used herein denotes single stranded nucleotide multimers of from about 10 to 100 nucleotides and up to 200 nucleotides in length.
- The term "sample" as used herein relates to a material or mixture of materials, typically, although not necessarily, in fluid form, containing one or more components of interest.
- The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g. wherein one or more of the hydroxyl groups are replaced with halogen atoms or aliphatic groups, or are functionalized as ethers, amines, or the like.
- The term "protein" as used herein means a polymer of amino acid residues linked together by peptide bonds. The term, as used herein, refers to proteins, polypeptides, and peptides of any size, structure, or function. Typically, however, a protein will be at least six amino acids long. Preferably, if the protein is a short peptide, it will be at least about 10 amino acid residues long. A protein may be naturally occurring, recombinant, or synthetic, or any combination of these. A protein may also be just a fragment of a naturally occurring protein or peptide. A protein may be a single molecule or may be a multi-molecular complex. The term protein may also apply to amino acid polymers in which one or more amino acid residues are an artificial chemical analogue of a corresponding naturally occurring amino acid. An amino acid polymer in which one or more amino acid residues is an "unnatural" amino acid, not corresponding to any naturally occurring amino acid, is also encompassed by the use of the term "protein" herein.

• The phrase "oligonucleotide bound to a surface of a solid support" refers to an oligonucleotide or mimetic thereof, such as PNA, that is immobilized on a surface of a solid substrate in a spot, where the substrate can have a variety of configurations, e.g. a sheet, bead, or other structure. In certain embodiments, the collections of features of oligonucleotides employed herein are present on a surface of the same planar support, e.g. in the form of an array.

• The term "array" encompasses the term "microarray" and "nanoarray". Arrays, as described in greater detail below, are generally made up of a number of distinct or different probes bound to the surface of a solid support, also referred to as substrate-immobilized probes. In one embodiment, the array of the application or invention is a homoarray, in which the array of the application or invention comprises probes of only one chemical type, i.e. only nucleic acid probes or only peptide or protein probes, etc. In another embodiment, the array of the application or invention is a heteroarray, in which the array of the application or invention comprises a mixture of types of probes, such as a mixture of nucleic acid probes and protein probes.

• The term "nanowell" applies to an area of nanometer dimensions in which probes are bound. The term "microwell" applies to an area of micrometer dimensions in which probes are bound.

• The term "NanoArrayDevice" or NAD applies to a feature (which may exist in many identical copies or different forms on a single chip) of micrometer dimensions. A NAD may comprise a set of nanowells or just a set of discrete areas (without wells but containing probes) that may themselves be or not be within a microwell.

• The term "common secondary ions" refers to those ions that are generated and analyzed in SIMS and that do NOT contain the rare, stable (or unstable) isotopes of their constituent elements or exogenous isotopes ($^{79}$Br, $^{81}$Br). Examples of common secondary ions include $^{12}$C, $^{14}$N, $^{32}$S, $^{80}$Br, $^{16}$O, etc.

• The term "rare secondary ions" refers to those ions that are generated and analyzed in SIMS and that DO contain at least one of the rare, stable (or unstable) isotopes of their constituent elements in the proportions used in the labeling of the probes and/or target molecules or at least one exogenous isotopes. Examples of rare secondary ions include $^{13}$C $^{15}$N, $^{13}$C$^{14}$N, $^{12}$C$^{15}$N, $^{13}$C$^{15}$N.

• The term "background common secondary ions" refers to those ions that are generated and analyzed in SIMS and that contain the rare, stable (or unstable) isotopes of their constituent elements in the naturally occurring proportions (as opposed to the proportions used in the labeling of the probes and/or target molecules). Examples of natural secondary ions in their natural proportions include $^{12}$C$^{14}$N$^-$, $^{13}$C$^{14}$N$^-$, $^{12}$C$^{15}$N$^-$ and $^{13}$C$^{15}$N$^-$ in the proportions 0.98538: 0.01096: 0.00362: 0.00004.

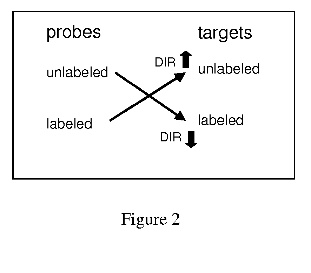

• The term "Detection Isotopic Ratio", or DIR, as applied to a discrete area or microwell or nanowell refers to the ratio of the number of the common secondary ions to the sum of the numbers of common plus at least one of the rare secondary ions obtained by SIMS or by related methods (e.g. $^{12}$C$^{14}$N$^-$/($^{12}$C$^{14}$N$^-$+$^{12}$C$^{15}$N$^-$) or $^{12}$C$^{14}$N$^-$($^{12}$C$^{14}$N$^-$+$^{13}$C$^{14}$N$^-$) or $^{12}$C$^{14}$N$^-$/($^{12}$C$^{14}$N$^-$ +$^{12}$C$^{15}$N$^-$+$^{13}$C$^{14}$N$^-$) or $^{12}$C$^{14}$N$^-$/($^{12}$C$^{14}$N$^-$ +$^{12}$C$^{15}$N$^-$ +$^{13}$C$^{14}$N$^-$+$^{13}$C$^{15}$N$^-$) or $^{12}$C$^-$/($^{12}$C$^-$ +$^{13}$C$^-$), etc.). Figure 2 shows the increase or decrease in the DIR depending on whether the probe or the target is labeled. (If both probe and target are labeled with, for example, different isotopes, the direction of change in the DIR must be worked out for each particular case).

• The term "exogenous isotope" refers to those isotopes that are not naturally contained in the biomolecules (probes and targets) but that have been added to these molecules by the user. The exogenous isotopes may be covalently linked to the biomolecules via standard chemistry or biochemistry. Alternatively, the exogenous isotopes may be covalently attached to a molecule that itself interacts strongly with either the probe or the target and that does not reduce significantly recognition of targets by probes. Examples of exogenous isotopes include: $^{79}$B and $^{81}$Br.

• The term "reference discrete area" refers to discrete areas, microwells or nanowells wherein probes (labeled or unlabeled) are not put into contact with the sample to be tested, or wherein there is no specific interaction between probe and target (i.e. the target is not present in the sample to be analyzed).


**THE APPLICATION OR INVENTION**


While carrying out the method of the application or invention with labeling the probes with $^{15}$N, the inventors found that coating the array with a hydrophilic polymer that does not contain the common secondary ions corresponding to the rare isotope used for labeling the probes, in this case nitrogen ($^{14}$N), gives an additional advantage to the method of the application or invention, as it prevents the non-specific adsorption of any hydrophobic molecules such as nucleic acids, peptides, or proteins present in the sample to be tested and thus minimizes the background.

Indeed, in order to minimize background, the person skilled in the art would have coated the array with bovine serum albumin (BSA) or casein as in conventional methods. However, the inventors found that coating the array with BSA or casein enhances the background in the method of the application or invention as it contains nitrogen. Thus, they found another means to minimize the background using a hydrophilic polymer that does not contain the common secondary ions corresponding to the rare isotope used for labeling the probes such as for example polyethylene glycol.

Without willing to be bound by a theory, the inventors state that the high number of water molecules bound to the hydrophilic polymer prevents the non specific binding of hydrophobic molecules, thus minimizing the background.

**Nature of supporting material and surface**

One object of the application is an array comprising a substantially planar substrate having a conducting surface and a number of discrete areas, which may or may not be in the form of wells, containing probes being labeled with at least one rare isotope (e.g. stable heavy or light isotope or unstable isotope) or with at least one exogenous isotope (e.g. $^{79}$Br, $^{81}$Br), wherein the array or discrete areas are coated with a hydrophilic polymer that does not contain the common secondary ions corresponding to the rare or exogenous isotope used for labeling the probes.

According to the application, the rare or exogenous isotope used for labeling the probes is the means for detecting the target in the sample to be tested; thus, the term "a hydrophilic polymer that does not contain the common secondary ions corresponding to the rare or exogenous isotope used for labeling the probes" is equivalent to "a hydrophilic polymer that does not contain the common secondary ions corresponding to the rare or exogenous isotope used for detecting the target".

The invention relates to an array comprising a planar substrate having a conducting surface and a number of discrete areas containing molecular probes being labeled with at least one rare heavy stable isotope selected in the group of $^{15}$N, $^{33}$S, $^{34}$S and $^{36}$S, or with at least one exogenous isotope selected in the group of $^{79}$Br and $^{81}$Br, wherein the array or the discrete areas are coated with a hydrophilic polymer that does not contain the common secondary ions $^{14}$N, $^{32}$S, $^{80}$Br, respectively, corresponding to the rare or exogenous isotope used for labeling the molecular probes.

The invention also relates to an array comprising a planar substrate having a conducting surface and a number of discrete areas containing molecular probes being labeled with at least one rare, stable, isotope selected in the group $^{2}$H, $^{13}$C, $^{15}$N, $^{17}$O, $^{18}$O, $^{33}$S, $^{34}$S and $^{36}$S, or with at least one rare, unstable, isotope selected in the group $^{3}$H and $^{14}$C or with at least one exogenous isotope selected in the group $^{79}$Br and $^{81}$Br, wherein the array or the discrete areas are coated with a hydrophilic polymer that does not contain the common secondary ions $^{1}$H, $^{12}$C, $^{14}$N, $^{16}$O, $^{32}$S, $^{80}$Br, respectively, corresponding to the rare or exogenous isotope used for labeling the molecular probes, but that contains at least one of said rare stable isotope or exogenous isotope used for labeling the molecular probes.

According to the application or invention, a (substantially) planar substrate having a conducting surface is a silicon wafer or any other solid or semi-solid surface made of gold, silver, aluminum, copper, platinum, palladium or other metal, or semiconductors such as GaAs, InP, or other material treated to make the surface conducting e.g. polymer material, polymer-coated material, superconducting material, ceramics, metal oxides, silicon oxide, etc.

In one embodiment of the application or invention, said (substantially) planar substrate having a conducting surface is compatible with SIMS.

In a preferred embodiment of the application or invention, said SIMS-compatible (substantially) planar substrate having a conducting surface is a silicon wafer.

According to the application or invention, the term "a polymer that does not contain the common secondary ions corresponding to the rare or exogenous isotope used for labeling the probes" refers for example to a polymer that does not contain nitrogen, in particular $^{14}$N, when the probes are labeled with $^{15}$N. In another example, it refers to a polymer that does not contain sulfur, in particular $^{32}$S, when the probes are labeled with $^{33}$S, $^{34}$S or $^{36}$S. Examples of common secondary ions of the application include $^{1}$H, $^{12}$C, $^{14}$N, $^{32}$S, $^{80}$Br, $^{16}$O...

In one embodiment of the application or invention, said hydrophilic polymer according to the application or invention (i.e. which does not contain the common secondary ions corresponding to the rare or exogenous isotope used for labeling the probes) contains at least one of said rare stable isotopes or exogenous isotopes used for labeling the probes. For example, said hydrophilic polymer may comprise $^{13}$C as carbon atoms only (and no $^{12}$C atom, as it corresponds to a common secondary ion), and/or $^{2}$H as hydrogen atoms only (and no $^{1}$H atom, as it corresponds to a common secondary ion), and/or $^{15}$N as nitrogen atoms only (and no $^{14}$N atom, as it corresponds to a common secondary ion).

According to one embodiment of the application or invention, said hydrophilic polymer that does not contain the common secondary ions corresponding to the rare or exogenous isotope used for labeling the probes can be selected in the group comprising monosaccharide, disaccharide, polysaccharide such as dextran, chemically modified polysaccharide such as hydroxymethylcellulose and synthetic hydrophilic polymer such as linear or branched polyvinylalcohols. Said hydrophilic polymer may also be a protein, which is natural or chemically modified, like for example protein A.

According to one embodiment of the application or invention, said synthetic hydrophilic polymer is polyethylene glycol. Polyethylene glycol presents the advantage in particular of not containing nitrogen or sulfur, and thus can be used preferably when probe labeling is done with $^{15}$N or $^{33}$S, $^{34}$S and $^{36}$S.

According to one embodiment of the application or invention, said hydrophilic polymer is coated onto the entire surface of the substantially planar substrate. According to this embodiment, the non-specific binding of molecules present in the sample to be tested is prevented on the whole surface, thus avoiding background and non-specific binding of the molecule to be tested.

## Discrete areas that are in the form of microwells

Another object of the application or invention is a microwell array being the microarray of the application or invention as described here above respectively, wherein the discrete areas are microwells that contain probes labeled with at least one rare, stable or unstable isotope as described above for the application or invention respectively. The microwells may be any shape, for example dots, lines, circles, squares or triangles, and may be arranged in any larger pattern, for example rows and columns, lattices, grids etc. These microwells contain probes labeled with at least one rare, stable or unstable isotope.

In one embodiment of the application or invention, the microwell array of the application or invention comprises from 10 to 100000 microwells, in another embodiment from 10 to 25000 microwells, in another embodiment from 100 to 10000 microwells and in another embodiment from 1000 to 5000 microwells.

In one embodiment of the application or invention, the shape and the size of the microwells are suitably determined to store a single cell in each microwell.

In one embodiment, each microwell has one of the dimensions length, width or diameter in the range from $1\,\mu$m to 1000 $\mu$m, in another embodiment from 1 $\mu$m to 500 $\mu$m, in another embodiment from 1 $\mu$m to 200 $\mu$m and in another embodiment from 1 to 100 $\mu$m.

In one embodiment, each microwell has a depth from 1 $\mu$m to 100 $\mu$m, in another embodiment from 1 $\mu$m to 50 $\mu$m, in another embodiment from 5 $\mu$m to 20 $\mu$m.

The distance between each microwell may be from 25 to 5000 $\mu$m, in another embodiment from 100 to 1000 $\mu$m and in another embodiment from 50 $\mu$m to 150 $\mu$m.

The microwell may be of any shape: for example it can be cylindrical, non-cylindrical such as a polyhedron with multiple faces (a parallelepiped, hexagonal column, octagonal column), an inverted cone, an inverted pyramid, or it may have a shape combining two or more of these shapes. For conical and parallelepiped shapes, the bottom of the microwell is normally flat, but curved surfaces (convex or concave) are also possible.

In one embodiment of the application or invention, the (substantially) planar conducting surface carrying the set of microwells may be shaped as a rectangular solid or a disc (although other shapes are possible), having a length of 1 cm, a width of 1 cm and a thickness of about 250 $\mu$m.

## Discrete areas in the form of nanowells

Another object of the application or invention is a nanowell array being the nanoarray of the application or invention as described here above respectively, wherein the discrete areas are nanowells that contain probes being labeled with at least one rare, stable or unstable isotope as described above for the application or invention respectively. The nanowells may be any shape, for example dots, lines, circles, squares or triangles, and may be arranged in any larger pattern, for example rows and columns, lattices, grids etc. These nanowells contain probes labeled with at least one rare, stable or unstable isotope.

In one embodiment of the application or invention, the nanowell array of the application or invention comprises from 10 to 100000 nanowells, in another embodiment from 10 to 25000 nanowells, in another embodiment from 100 to 10000 nanowells and in another embodiment from 1000 to 5000 nanowells.

In one embodiment, each nanowell has one of the dimensions length, width or diameter being from 1 nm to 1000 nm, in another embodiment from 5 nm to 500 nm, in another embodiment from 10 nm to 200 nm and in another embodiment from 50 to 100 nm.

In one embodiment, each nanowell has a depth from 1 nm to 100 nm, in another embodiment from 1 nm to 50 nm, in another embodiment from 5 nm to 20 nm.

The distance between each nanowell may be from 10 to 1000 nm, in another embodiment from 50 to 500 nm and in another embodiment from 100 nm to 200 nm.

The nanowell may be of any shape: for example it can be cylindrical, noncylindrical such as a polyhedron with multiple faces (a parallelepiped, hexagonal column, octagonal column), an inverted cone, an inverted pyramid, or it may have a shape combining two or more of these shapes. For conical and parallelepiped shapes, the bottom of the nanowell is normally flat, but curved surfaces (convex or concave) are also possible.

In one embodiment of the application or invention, the (substantially) planar conducting surface carrying the set of nanowells may be shaped as a rectangular solid or a disc (although other shapes are possible), having a length of 1 cm, a width of 1 cm and a thickness of about 250 $\mu$m.

## Discrete areas that are not in the form of wells

Another object of the application or invention is an array being an arrangement of a set of discrete areas, or pattern units, forming a larger pattern on a substrate. The discrete areas or pattern units may be any shape, for example dots, lines, circles, squares or triangles, and may be arranged in any larger pattern, for example rows and columns, lattices, grids etc. These discrete areas contain probes labeled with at least one rare, stable (or unstable) isotope as described above for the application or invention respectively.

In one embodiment of the application or invention, the array of the application or invention comprises from 10 to 100000 discrete areas, in another embodiment from 10 to 25000 discrete areas, in another embodiment from 100 to 10000 discrete areas and in another embodiment from 1000 to 5000 discrete areas.

In one embodiment of the application or invention, the array of the application or invention is a microarray. In this embodiment, each discrete area has one of the dimensions length, width or diameter being from 1 $\mu$m to 1000 $\mu$m, in another embodiment from 5 $\mu$m to 500 $\mu$m, in another embodiment from 10 $\mu$m to 200 $\mu$m and in another embodiment from 50 to 100 $\mu$m. The distance between each discrete area may be from 10 to 1000 $\mu$m, in another embodiment from 50 to 500 $\mu$m and in another embodiment from 100 $\mu$m to 200 $\mu$m.

In one embodiment of the application or invention, the array of the application or invention is a nanoarray. In this embodiment each discrete area has one of the dimensions length, width or diameter being from 1 nm to 1000 nm, in another embodiment from 5 nm to 500 nm, in another embodiment from 10 nm to 200 nm and in another embodiment from 50 to 100 nm. The distance between each discrete area may be from 10 to 1000 nm, in another embodiment from 50 nm to 500 nm and in another embodiment from 100 $\mu$m to 200 nm.

In one embodiment of the application or invention, the membrane or silicon wafer carrying the set(s) of discrete areas may be shaped as a rectangular solid or a disc (although other shapes are possible), having for example a length of 1 cm, a width of 1 cm and a thickness of about 250 um.

### Probes

#### *Chemical nature and isotopic composition of probes*

In this embodiment, the probes, which bind to targets (usually the biomolecules that constitute cells but even viruses, organelles or cells themselves), may be made of any molecules (biological or non-biological) such as nucleic acids (oligonucleotides, DNA, RNA, PNA, aptamers), peptides or proteins (antibodies, enzymes), ligands (an antigen, enzyme substrate, receptor or ligand for the receptor), glycans, lipids, polyamines, molecularly imprinted polymers, phages, viruses, or combination of these molecules.

Preferably, the probes are molecules which are able to specifically bind to their target(s) molecule(s) (for example via a non covalent bond), or specifically interact with their target(s) molecule(s) (for example via hybridization, as is the case for nucleic acids).

According to the application or invention, the probes contain at least one rare, stable or unstable isotope:

in one embodiment, probes contain at least one heavy stable isotope such as $^2$H, $^{13}$C, $^{15}$N, $^{17}$O, $^{18}$O, $^{33}$S, $^{34}$S and $^{36}$S. in another embodiment, probes contain at least one unstable isotope such as $^3$H and $^{14}$C, in another embodiment, probes contain at least one exogenous isotope such as $^{79}$Br and $^{81}$Br.

#### *Attachment or grafting of probes*

The attachment or grafting of probes to the array is achieved by techniques well-known in the art. The probes may be adsorbed, physisorbed, chemisorbed, or covalently attached to the arrays. Lithography printing may also be used to allow probes to be transferred and adsorbed directly or indirectly to surfaces in a patterned fashion.

For example, attachment of probes may be achieved by introducing functional groups onto the surface for chemical reaction between the surface and the probe to be grafted.

The carboxyl group (COOH) is one of the best-known functional groups for grafting. Chemical bonds are produced between amino-groups from proteins and carboxyl functional groups. Acrylic acid or copolymerised vinylsilane and maleic anhydride acid can also be used to generate silicon-COOH substrates that act as spacers to graft proteins onto the surface using e.g. 1-ethyl-3-(3-dimethylaminopropyl) carbodiimide hydrochloride.

In one embodiment of the application or invention, the attachment or grafting of probes to the array is achieved via functionalization of the hydrophilic polymer coated onto the array.

For example, in one embodiment of the application or invention, the array is coated with polyethylene glycol which is functionalized with N-hydroxysuccimide (NHS) to allow the grafting of probes. This grafting presents the advantage of being very simple to carry out.

#### *Identification of probes*

In one embodiment in which a diversity of different probes is attached to the array, the probes are identified via the position or coordinates of the discrete area containing the probes.

**Nature and origin of samples**

According to the method of the application or invention, the sample to be tested may be isolated from cells, tissue, organ, body fluid such as for instance sera, plasma, seminal fluid, synovial fluid, cerebrospinal fluid, blood or urine, a cell culture, water such as sewage water, freshwater, marine coastal water, ground water...

According to the method of the application or invention, the sample to be tested may comprise nucleic acids (oligonucleotides, DNA, RNA, PNA), peptides or proteins (antibodies, enzymes), ligand (an antigen, enzyme substrate, receptor or ligand for the receptor), glycans, lipids, polyamines, phages, viruses or a combination thereof. Thus the biomolecules to be detected may be nucleic acids (oligonucleotides, DNA, RNA, PNA), peptides or proteins (antibodies, enzymes, prions), ligand (an antigen, enzyme substrate, receptor or ligand for the receptor), glycans, lipids, polyamines, phages, viruses or a combination thereof.

**Labeling of probes**

According to the method of the application or invention, the probes or the targets, i.e. the biomolecules present in the sample(s) to be tested, are labeled with rare, stable or unstable isotopes or exogenous isotopes.

In one embodiment, probes or targets may be labeled with at least one rare, stable isotope such as $^2$H, $^{13}$C, $^{15}$N, $^{17}$O, $^{18}$O, $^{33}$S, $^{34}$S and $^{36}$S.

In another embodiment, probes or targets may be labeled with at least one unstable isotope such as $^3$H and $^{14}$C.

In another embodiment, probes or targets may be labeled with at least one exogenous isotope such as $^{79}$Br and $^{81}$Br. Labeled probes can be obtained by two techniques, in vivo and in vitro:

In the case of in vitro labeling, 1) the polymerase chain reaction is used to produce labeled oligonucleotides, 2) peptide synthesis is used to produce labeled peptides, 3) in vitro transcription/translation is used to produce labeled RNA and labeled proteins, 4) reverse transcription is used to produce labeled cDNA, 5) chemical synthesis is used to produce labeled PNA.

In the case of in vivo labeling, either prokaryotic or eukaryotic cells are grown on media containing nutrients (such as $NH_4Cl$, glucose and amino acids) labeled with combinations of $^2$H, $^{13}$C, $^{15}$N, $^{17}$O, $^{18}$O, $^{33}$S, $^{34}$S and $^{36}$S or with combinations of $^3$H $^{14}$C, or other unstable isotopes with long half-lives. These cells thus produce labeled probes (such as antibodies or bacteriophage or nucleic acids or proteins or sugars or other cellular constituents).

Where the targets, i.e. the biomolecules present in the sample(s) to be tested, are to be labeled, the cells are generally grown on media containing nutrients (such as $NH_4Cl$, glucose and amino acids) labeled with combinations of $^2$H, $^{13}$C, $^{15}$N, $^{17}$O, $^{18}$O, $^{33}$S, $^{34}$S and $^{36}$S or with combinations of $^3$H and $^{14}$C. These cells thus produce labeled biomolecules.

**Contact between sample and array**

In one embodiment of the method of the application or invention, the sample to be tested is put in contact with the array (microarray or nanoarray) containing a number of diverse probes. In another embodiment, where a number of samples is to be tested, each sample is put in contact with one or more microwells of a microwell array.

In another embodiment, where the number and diversity of samples to be tested corresponds to the number and diversity of single cells, the contents of each single cell, obtained via an appropriate method, is put into contact with one microwell of a microwell array.

**Conditions of interaction between probe and target**

The sample(s) is/are then contacted with the array under conditions that allow the probes present onto the array to interact with the target biomolecules.

The binding of probes to their targets is then performed in a variety of buffers from which, typically, the common $^{12}$C and $^{14}$N isotopes are absent (such as Phosphate Buffered Saline).

After careful washing with pure water (preferably at low temperature to limit the dissociation of probes from their targets) to eliminate salts (which can form crystals at the drying step), the unbound molecules and the big cellular debris, the array is dried in a dust-free atmosphere either in an oven under vacuum or by freeze-drying.

**Detection by SIMS and other related techniques**

According to the method of the application or invention, counting the numbers of rare and common secondary ions so as to obtain the Detection Isotopic Ratio allows detection of the duplexes probe/target.

In one embodiment, the duplexes probe/target are detected by Dynamic Secondary Ion Mass Spectrometry (D-SIMS). In another embodiment, the duplexes probe/target are detected by Time-of-Flight Secondary Ion Mass Spectrometry (TOF-SIMS).

Secondary Ion Mass Spectrometry (SIMS) allows the analysis of the surface composition of inorganic and organic materials based on mass spectral analysis of secondary ions extracted from the surface (1nm - 1$\mu$m depth) of a solid sample under the impact of an energetic beam of primary ions. Molecules are fragmented by the primary ion beam in D-SIMS either totally into their constituent atoms by dynamic D-SIMS or partially into molecular fragments such as amino acids by ToF-SIMS. SIMS-related technologies such as Laser SNMS may also be used to detect probe/target interactions.

In one embodiment of the method of the application or invention, the probes that are attached to the array are labeled with rare, stable or unstable isotopes or exogenous isotopes. In this embodiment, the sample(s) to be tested have biomolecules (targets) that only contain these rare isotopes in their natural proportions (or even lower than these proportions).

The presence of an interaction probe/target is determined by the comparison of the Detection Isotopic Ratio obtained from each discrete area, microwell or nanowell to the background Detection Isotopic Ratio obtained from the reference discrete area, microwell or nanowell or from discrete areas in which there is no specific interaction between labeled probe and target (i.e. the target is not present in the sample to be analyzed) or in which there is a specific interaction between unlabeled probe and unlabeled target.

Therefore, in the case where probes are labeled and targets are unlabeled, the presence of an interaction probe/target is revealed if the Detection Isotopic Ratio obtained from one discrete area, microwell or nanowell is significantly superior to the Detection Isotopic Ratio obtained from the reference discrete area, microwell or nanowell. For example, in the case where probes are labeled with $^{13}$C, the presence of an interaction probe/target is revealed if the Detection Isotopic Ratio of $^{12}$C/($^{12}$C+ $^{13}$C) (or alternatively $^{12}$C$^{14}$N/($^{12}$C$^{14}$N + $^{12}$C$^{15}$N) ) obtained from one discrete area, microwell or nanowell is superior to the Detection Isotopic Ratio obtained from the reference discrete area, microwell or nanowell, for the same instrumental settings.

Therefore, in the case where probes are unlabeled and targets are labeled, the presence of an interaction probe/target is revealed if the Detection Isotopic Ratio obtained from one discrete area, microwell or nanowell is significantly inferior to the Detection Isotopic Ratio obtained from the reference discrete area, microwell or nanowell. For example, in the case where probes are labeled with $^{13}$C, the presence of an interaction probe/target is revealed if the ratio of $^{12}$C/($^{12}$C+ $^{13}$C) secondary ions (or alternatively $^{12}$C$^{14}$N/($^{12}$C$^{14}$N + $^{12}$C$^{1s}$N) ) obtained from one discrete area, microwell or nanowell is inferior to this ratio of secondary ions obtained from the reference discrete area, microwell or nanowell, for the same instrumental settings.

Therefore, in the case where both probes and targets are labeled with the same isotope/isotopes, the presence of an interaction probe/target is revealed if the Detection Isotopic Ratio obtained from one discrete area, microwell or nanowell is significantly different from the Detection Isotopic Ratio obtained from the reference discrete area, microwell or nanowell.

According to the application or invention, the probes or targets may also be labeled with more than one rare, stable or unstable isotopes or exogenous isotopes of their constituent elements. For example, probes or targets may be labeled with $^{13}$C and $^{15}$N. Multiple labeling such as $^{13}$C$^{15}$N of the probes is particularly useful to minimize the background.

According to the application or invention, where the targets are labeled with rare, stable (or unstable) isotopes, various experimental conditions (for example, with and without drug treatments) may be differentiated by a differential labeling of each condition.

**The method of detection and quantification**

One object of the application is a method for detecting and quantifying in at least one sample the presence or absence of at least one biomolecule, comprising:

    (a) putting at least one of said samples into contact with an array of the application under conditions that allow the probes present on the array to interact with the target biomolecules, the probes present on the array or the biomolecules present in the sample being labeled with at least one rare, stable (or unstable) isotope,
    (b) washing and drying the array,
    (c) detecting and counting by SIMS the common secondary ions along with the corresponding rare secondary ions.

The invention relates to an *in vitro* method for detecting and quantifying in at least one sample the presence or absence of at least one biomolecule, comprising:

(a) contacting said at least one sample with an array according to the invention,

(b) washing and drying the array,

(c) detecting and counting by SIMS the common secondary ions along with the corresponding rare secondary ions.

This allows the determination of the DIR.

The DIR obtained from each discrete area, microwell or nanowell is then compared to the DIR obtained from the reference discrete area, microwell or nanowell; a significant change of the DIR (see Figure 2) is an evidence for the presence (detection) of an interaction probe/target. Moreover, the precise determination of the DIR allows quantifying the number of interactions between probe and target .The precise determination of the DIR is obtained after sputtering the maximum quantity of material so as to obtain good counting statistics of both the common and the rare secondary ions. To calculate the number of targets interacting with the probes per discrete area (microwell or nanowell or indeed a sample unit area within the discrete area) it is necessary to know: a) the density of the probes on the surface of the discrete area and b) the number of atoms of each isotope of interest in the both individual probe and individual target. This knowledge then allows the DIR to be plotted as a function of the number of targets per discrete area.

### APPLICATIONS OF THE PATENT APPLICATION OR OF THE INVENTION

According to another embodiment, said method for detecting and quantifying in at least one sample the presence or absence of at least one biomolecule, allows the determination of a molecular atlas of said sample, which means the determination of the transcriptome, proteome, lipidome, metabolome, glycome or interactome of said sample, or allows the determination of biomolecules and their interactions in a single cell.

### Application to molecular atlas

According to one embodiment of the application or invention, the method of the application or invention for detecting and quantifying in a sample the presence or absence of at least one biomolecule, is intended for providing a molecular atlas of said sample.

In one embodiment, the diversity of biomolecules to be detected and quantified is genomic sequences, allowing the determination of genomic variation in said sample.

In another embodiment, the diversity of biomolecules to be detected and quantified is RNA, allowing the determination of the transcriptome of said sample.

In another embodiment, the diversity of biomolecules to be detected and quantified is proteins, allowing the determination of the proteome of said sample.

In another embodiment, the diversity of biomolecules to be detected and quantified is lipids, allowing the determination of the lipidome of said sample.

In another embodiment, the diversity of biomolecules to be detected and quantified is metabolites, allowing the determination of the metabolome of said sample.

In another embodiment, the diversity of biomolecules to be detected and quantified is glycosylated proteins, allowing the determination of the glycome of said sample.

In another embodiment, the diversity of biomolecules to be detected and quantified is proteins that interact with at least one specific probe, allowing the determination of the interactome of said sample.

### Application to single cell

According to another embodiment of the application or invention, the method of the application or invention for detecting and quantifying at a single cell level the presence or absence of at least one biomolecule, is intended for providing a molecular atlas of a single cell level.

In one embodiment, the diversity of biomolecules to be detected and quantified is genomic sequences, allowing the determination of genomic variation at the single cell level.

In another embodiment, the diversity of biomolecules to be detected and quantified is RNA, allowing the determination of the transcriptome at the single cell level.

In another embodiment, the diversity of biomolecules to be detected and quantified is proteins, allowing the determination of the proteome at the single cell level.

In another embodiment, the diversity of biomolecules to be detected and quantified is lipids, allowing the determination of the lipidome at the single cell level.

In another embodiment, the diversity of biomolecules to be detected and quantified is metabolites, allowing the determination of the metabolome at the single cell level.

In another embodiment, the diversity of biomolecules to be detected and quantified is glycosylated proteins, allowing the determination of the glycome at the single cell level.

In another embodiment, the diversity of biomolecules to be detected and quantified is proteins that interact with at least one specific probe, allowing the determination of the interactome at the single cell level.

**Application to disease**

Another object of the application or invention is the use of said method for detecting and quantifying at least one biomolecule in a sample or at a single cell level for:

- predicting a predisposition to a disease, or for diagnosing a disease in a subject,
- screening therapeutic agents,
- monitoring the efficacy of a therapeutic agent administrated to a subject in order to treat a disease.

Another object of the application or invention is a method for predicting a predisposition to a disease, or for diagnosing a disease in a subject. This comprises:

(a) putting at least one sample isolated from said subject in contact with an array of the application or invention under conditions that allow the probes present onto the array to interact with the target biomolecules known to be associated with or differentially expressed in said disease, the probes present on the array being labelled with at least one rare, stable (or unstable) isotope as described above for the application or invention respectively,
(b) washing and drying the array,
(c) detecting and counting by SIMS the common secondary ions along with the corresponding rare secondary ions, wherein the number of interactions between probe and target is quantified by the comparison of the DIR obtained from each discrete area, microwell or nanowell to the DIR obtained from the reference discrete area, microwell or nanowell or from discrete areas in which there is no specific interaction between probe and target (i.e. the target is not present in the sample to be analyzed) or in which there is a specific interaction between unlabeled probe and unlabeled target,
(d) comparing said pattern of expression to a standard pattern, wherein the presence/absence or the increase/decrease of said at least one biomolecule known to be associated with or differentially expressed in said disease is indicative of a likelihood to develop a disease or of the presence of a disease.

The method of the invention is an *in vitro* method.
In one embodiment, the subject is a mammal. In a preferred embodiment, the subject is a human being.
According to the method of the application or invention, the sample to be tested may be isolated from cells, tissue, organ, or body fluid such as for instance sera, plasma, seminal fluid, synovial fluid, cerebrospinal fluid, blood or urine, from the subject.
The sample may be derived from diseased cells or tissues. For example, the cells or tissues may be infected by a pathogen such as HIV, influenza, malaria, hepatitis, cytomegalovirus, herpes simplex virus. In one embodiment, the cells or tissues are infected by a viral or a bacterial pathogen. In another embodiment, the disease is cancer. In another embodiment, the disease is a neurodegenerative disease such as Parkinson, Alzheimer or Multiple Sclerosis.
In one embodiment of the application or invention, said method is intended to predict a predisposition to a cancer, or for diagnosing a cancer in a subject.
In another embodiment of the application or invention, said method is intended to predict a predisposition or to diagnose a bacterial disease.
In another embodiment of the application or invention, said method is intended to predict a predisposition or to diagnose a viral disease.
The method of the invention is an *in vitro* method.
Another object of the application or invention is a method for screening therapeutic agents, comprising:

(a) cultivating cells with at least one therapeutic agent of interest,
(b) putting at least one sample isolated from said culture in contact with an array of the application or invention under conditions that allow the probes present onto the array to interact with the target biomolecules, the probes present on the array being labelled with at least one heavy or unstable isotope as described above for the application or invention respectively,
(c) washing and drying the array,
(d) detecting and counting by SIMS the common secondary ions along with the corresponding rare secondary ions, wherein the number of interactions between probe and target is quantified by the comparison of the DIR obtained from each discrete area, microwell or nanowell to the DIR obtained from the reference discrete area, microwell or nanowell or from discrete areas in which there is no specific interaction between probe and target (i.e. the target is not present in the sample to be analyzed) or in which there is a specific interaction between unlabeled probe and unlabeled target,

(e) comparing said pattern of expression to a standard pattern, wherein the increase or decrease of certain biomolecules is indicative of the efficacy of said therapeutic agent.

The method of the invention is an *in vitro* method.

Another object of the application or invention is a method for monitoring efficacy of a therapeutic agent administrated to a subject to treat a disease, said method comprising:

(a) putting at least one sample isolated from said subject in contact with an array of the application or invention under conditions that allow the probes present onto the array to interact with the target biomolecules, the probes present on the array being labelled with at least one heavy or unstable isotope as described above for the application or invention respectively,

(b) washing and drying the array,

(c) detecting and counting by SIMS the common secondary ions along with the corresponding rare secondary ions, wherein the number of interactions between probe and target is quantified by the comparison of the DIR obtained from each discrete area, microwell or nanowell to the DIR obtained from the reference discrete area, microwell or nanowell or from discrete areas in which there is no specific interaction between probe and target (i.e. the target is not present in the sample to be analyzed) or in which there is a specific interaction between unlabeled probe and unlabeled target,

(d) obtaining a pattern of expression from said sample of at least one biomolecule at different times during the treatment of the subject,

(e) comparing said patterns of expression to a standard pattern, wherein the increase or decrease of said biomolecules known to be associated with or differentially expressed in said disease is indicative of the efficacy of said therapeutic agent for treating the subject.

The method of the invention is an *in vitro* method.

## EXAMPLES

In the following description, all experiments for which no detailed protocol is given are performed according to standard protocol.

The following examples are included to demonstrate preferred embodiments of the application or invention. It should be appreciated by those of skill in the art that the techniques disclosed in the examples which follow represent techniques discovered by the inventor to function well in the practice of the application or invention, and thus can be considered to constitute preferred modes for its practice.

## EXPERIMENT 1:

This experiment shows that the SIMS technique allows a quantitative detection of the proportion of the $^{13}C$ isotope in a mixture containing isotopically labeled proteins and a different amount of unlabeled molecules.

## Isotopic labeling and dilution

Isotope-labeled molecules are generally supplied in a buffer containing protectants and preservatives 12 14 such as glycerol (a source of C) and sodium azide (a source of N). These agents are used to preserve dialysis membranes (as supplied by Millipore). The aim of the following experiments is to determine the conditions of dialysis needed to remove them.

Proteins were extracted from *E. coli* grown in a minimal medium in which the only available nitrogen 15 was N. Using SIMS, the isotopic fraction of these proteins was found to be 97.8%

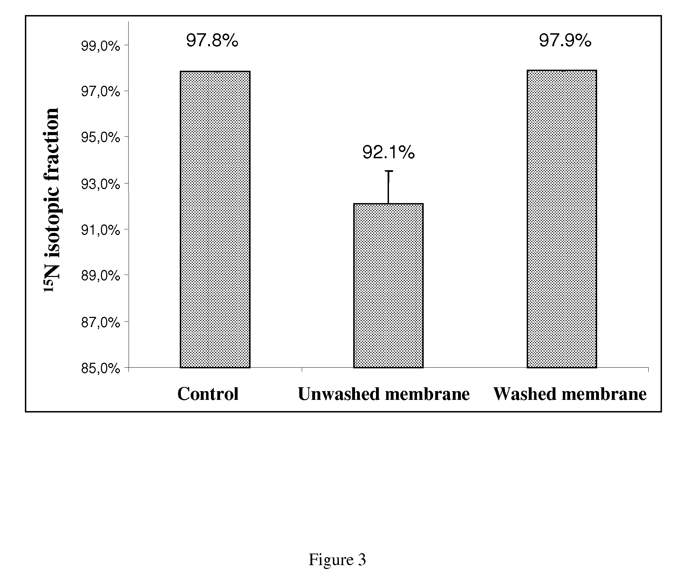

$\pm$ 0.02%. 20$\mu$g of these labeled proteins were dialysed for 6 hours in water on Millipore dialysis membranes which either were unwashed or had been washed previously. Figure 3 shows the isotopic fraction of $^{15}N$ in the dialyzed solutions.

The level of the isotopic fraction of the washed membrane was similar to that of the control (97.9% $\pm$ 0.02%) whilst that of the unwashed membrane (containing contaminant nitrogen) was lower (92.1% $\pm$ 1.4%). Hence, prewashing the dialysis membrane restored the initial isotopic fraction.

Proteins were also extracted from bacteria grown in a medium in which the only available carbon was $^{13}C$. The

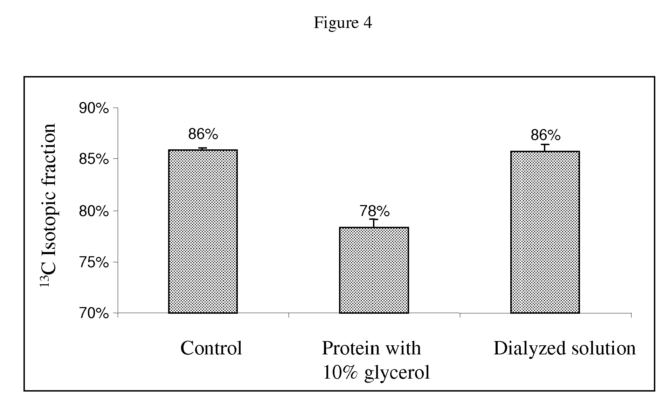

isotopic fraction of these proteins as measured by SIMS was 86% ± 0.5%. These proteins were then dissolved in water and a second solution of them was made to a final concentration of 10% glycerol. Figure 4 shows the isotopic fraction of $^{13}$C in these solutions.

The addition of glycerol reduced the isotopic fraction to 78% ± 2% but the initial fraction (86% ± 2%) was recovered by the subsequent dialysis which eliminated the glycerol.

### EXPERIMENT 2:

This experiment shows that the isotopically labeled oligonucleotide (probes) can be prepared using the PCR technique and an unconventional buffer that does not contain nitrogen compounds (which, as contaminants, might perturb the DIR measurement described in the section "Definitions").

### Use of a borax buffer to reduce nitrogen contamination

### Introduction

Many methods in biology use Tris-based buffers. This organic molecule is an excellent buffer and, moreover, does not lead to the precipitation of calcium or magnesium salts. For our method, however, Tris has the disadvantage of containing $^{14}$N and of having a strong affinity for DNA (and other macromolecules). This disadvantage arises because this $^{14}$N is an important contaminant in our method which depends on measuring accurately the $^{15}$N/($^{14}$N+$^{15}$N) ratio resulting from the hybridization of a $^{15}$N probe with a $^{14}$N target.

The two approaches to making DNA without contaminant nitrogen are either i) using Tris in the buffers and then removing it once the DNA has been made or ii) using Tris-free buffers. Since the first method entails purification methods that are time-consuming, wasteful and expensive, we adopted the second solution to provide the right pH and concentration conditions for PCR (polymerase chain reaction). Although, to our knowledge, no literature describes the use of borax buffers for PCR, we used a borax buffer, an inorganic buffer that does not contain nitrogen and that maintains the same pH values as Tris buffers.

### Material and methods

PCR method

| chemicals | C init | volume | C final |
|---|---|---|---|
| Borax Buffer | 0.08 M | **12.5** | 20mM |
| KCl | 1M | **2.5** | 50 mM |
| MgC12 | 25 mM | **4** | 2 mM |
| dNTP | 10 mM | **2** | 0.8 mM |
| Taq Polymerase | 1 U/ $\mu$L | **2.5** | 0.05 U/$\mu$L |
| Primer S | 100 $\mu$M | **0.5** | 1 $\mu$M |
| Primer AS | 100 $\mu$M | **0.5** | 1 $\mu$M |
| DNA Sample | - | **1** | |
| Water | - | **24.5** | |
| Total | | **50** | |

A 200 bp sequence of DNA from the PML gene was amplified. The template itself was made using only one round of amplification (to avoid error generation). Either 20 cycles of PCR were performed to determine quantitatively the effect of the borax buffer (and limit error generation) or 60 cycles were performed to consume the limiting substrate and obtain maximal amplification. The amplicons were analyzed by electrophoresis on 1% agarose gels. The results below are shown for a Tris buffer and for a borax buffer.

**Results**

In the case of 20 cycles of amplification, the borax buffer gives half the yield of the Tris buffer and, significantly, the borax buffer did not generate amplicons of different sizes. This means that the polymerase continued replication to the end of the templates. In the case of 60 cycles of amplification, both Tris and borax buffers gave similar results.

**Conclusion**

A borax buffer is perfectly suitable for reactions such as PCR amplification and hence for the synthesis of oligonucleotide probes. Hence this buffer can be used to avoid contaminant nitrogen as required in the SIMS analyses proposed here.

**EXPERIMENT 3:**

Protein arrays were prepared for detection by SIMS.

Wafers were coated with PEG functionalized with N Hydroxy Succinimide (NHS) to allow the anti-CD34 antibody enriched in $^{15}$N to be linked to the PEG coat of a silicon wafer. Three identical anti-CD34 wafers were prepared. The first wafer was incubated with the target protein (Wtap for target protein), CD34 whilst, as negative controls, the second wafer was incubated with BSA (Wbsa for BSA) and the third wafer was incubated in the same solution but without protein (Wabo for antibody only).

The three wafers were then analyzed using a NanoSIMS 50.

***Material and Methods :***

*Linking antibody to silicon wafer*

1$\mu$L of a solution of 0.22 $\mu$g/$\mu$L of an anti-CD34 antibody (supplied by Biosynergy, Cambridge UK) enriched with $^{15}$N (Detection Isotopic Ratio was 58 $\pm$ 11 %) was deposited on a silicon wafer coated with PEG functionalized with NHS (Microsurfaces Inc., Minneapolis, USA). After 1h at room temperature (25°C), the surface was washed 5 x for 5 minutes with a solution of PBS pH 7.4 Tween-20 (0.1% v/v) then for 30 minutes with a solution of PBS pH 8 Tween-20 (0.1% v/v) and finally for 5 minutes with water (milli-Q).

*Incubation with target protein (CD34) and with control (BSA)*

To be sure to saturate the antibodies with the target protein, the ratio of target protein to antibody (linked to the wafer in a functional orientation for target binding) was chosen to be 100:1. We estimated from the data of the supplier (Proteomics 2005, 5, 416-419) that the surface concentration of the antibody in a functional orientation was of the order of $10^{12}$ proteins cm$^{-2}$ and hence in the area (spot) on the wafer to which the antibody was linked (0.02 cm$^{-2}$) there were 2 x $10^{10}$ functional antibodies. We therefore chose to use a solution that would provide 0.1 $\mu$g of CD34 (Mr 35 kDa) which contains around 2 x $10^{12}$ proteins.

10 $\mu$L of a solution of PBS-Tween-20 pH 7.4 (Ab + no protein), or of a solution of PBS-Tween-20 pH 7.4 containing 0.01 $\mu$g/$\mu$L of the recombinant protein CD-34 (Assay Designs, Ann Arbor, USA) (sample Ab + CD34), or of a solution of PBS-Tween-20 pH 7.4 containing 0.01 $\mu$g/$\mu$L of BSA (Sigma) (Ab + BSA) were put on the surface of the wafer. After incubation for 1h at room temperature, the surface of the wafer was washed 4 x for 5 minutes with a solution of PBS pH 7.4 Tween-20 (0.1% v/v) then 2 x for 5 minutes with water (milli-Q). The wafers were then dried under a vacuum and covered with gold for 60 seconds using a Cressington sputter coater 108 auto.

*SIMS analysis and calculation of the isotopic fraction*

The surface of the wafers was analyzed using a NanoSIMS 50 in the imaging mode (field: 20 x 20 $\mu$m$^2$, 128 x 128 pixels, 10 ms/pixel diaphragm $D_{1}$). This entailed sampling in four to five different places within the area where the antibody was linked; 12 successive scans were performed at each of these places. These scans were performed with settings that were estimated to give a sputtering rate of 0.3 nm/scan after removal of the gold layer which is sputtered out rapidly (around 1.5 nm/scan). The sum of the average number of counts of secondary ions $^{12}$C$^{14}$N, ñ($^{12}$C$^{14}$N ), and of $^{12}$C$^{15}$N, ñ($^{12}$C$^{15}$N), of scans 2 to 12 were used to obtain the Detection Isotopic Ratio in each of the 3 samples using the formula:

$$\text{Detection Isotopic Ratio} = (\sum \tilde{n}^{12}C^{15}N) / (\sum \tilde{n}^{12}C^{15}N + \sum \tilde{n}^{12}C^{14}N).$$

*Results:*

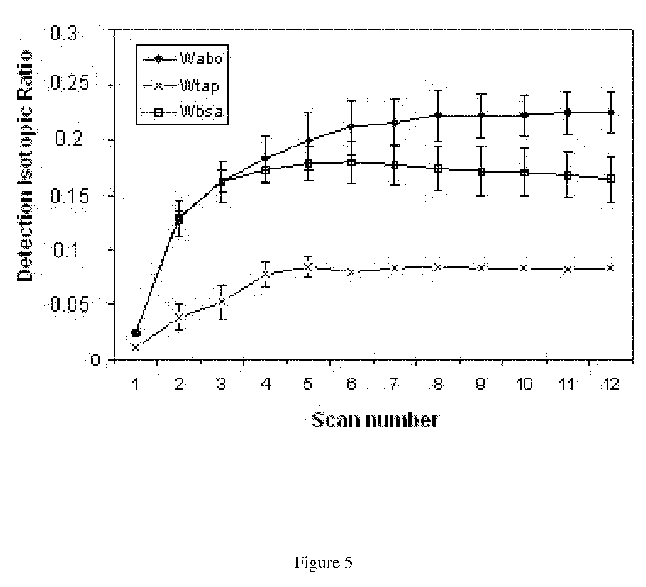

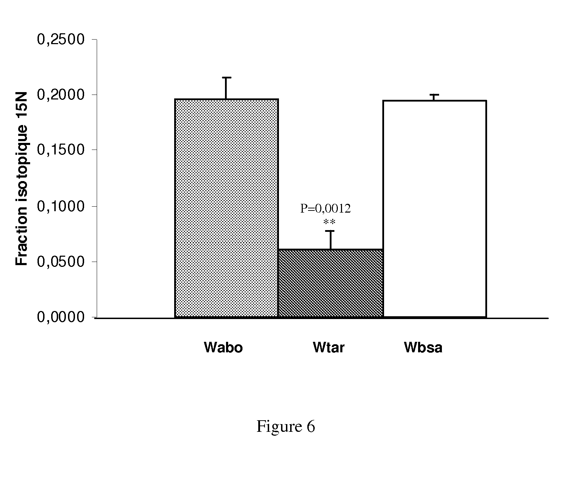

The Detection Isotopic Ratio obtained from the wafer with antibody only (Wabo) shows a monotonous increase from a value close to that of the natural isotopic ratio of $^{15}N$ (0.0037) to a plateau of 0.22 (Figure 5, Wabo). This shows that nitrogen-containing contaminants were present in the gold surface. These contaminants were also found in the scans, which explains why the value of 0.22 is substantially lower than that expected for the $^{15}N$ isotopic ratio of the antibody. The Detection Isotopic ratio obtained from the wafer with both antibody and target protein, CD34, has a plateau of 0.083 (Figure 5, Wtap). To improve the statistics of the comparison between the Detection Isotopic Ratios of the wafers with and without target protein, we summed the counts from scans 2 to 12 (Figure 6). The Detection Isotopic Ratios so obtained showed a highly significant difference between the wafer with the antibody+target protein (Wtap = $0.06\pm0.03$) and the two controls (Wabo = $0.20\pm0.04$ and Wbsa = $0.19\pm0.01$). Therefore $R_{DIR}$, the (Detection Isotopic Ratio Wtap)/(Detection Isotopic Ratio Wabo), equals 0.30. If we assume that each antibody binds two target proteins and that antibodies and targets are entirely sputtered, we calculate that the $R_{DIR}$ should be 0.66 in the absence of nitrogen-containing contaminants whilst the $R_{DIR}$ should tend to 1 with increasing amounts of such contaminants. The reason is that, given the rate of sputtering, a total of only 3.5-4 nm were sputtered in the scans of the wafer. In the case of the wafer with the antibodies plus target proteins, 3.5-4 nm corresponds to the sputtering being mainly of the $^{14}N$ target proteins rather than of the $^{15}N$-labeled antibodies linked to the coating on the wafer. Hence the Detection Isotopic Ratio Wtap measured in these scans is lower than would be measured if the entire region would sputtered. In other words, the method allows detection of the binding of probe to target but a complete sputtering of the target-probe region is needed to obtain an accurate and quantitative measure of the proportion of targets bound to probes.

The possibility of using the capacity of the NanoSIMS 50 to obtain successive scans opens up the possibility of obtaining a 3 dimensional image of the structure of regions containing probes and targets and contaminants. Clearly, this capacity may then used to further refine the technique.

Finally, the control with BSA has a Detection Isotopic Ratio similar to that of the control without protein. This shows that the materials and methods we used for incubating target proteins with the antibodies and for subsequent washing steps do not result in the non-specific adsorption of proteins to the surface.

In particular, the coating of wafers with polyethylene glycol did prevent non-specific adsorption of proteins to the surface, while coating of wafers with BSA or casein resulted in high background (data not shown).

When the antibody linked to the wafer is incubated with the target protein (Wtar) there is a significant drop in the Detection Isotopic Ratio with respect to $^{15}N$ in comparison with the negative controls (Wabo, absence of target protein; Wbsa, presence of Bovine Serum Albumin). This drop is significant as shown by the P value of 0.0012 (i.e. a certainty of 99.88%).

**EXPERIMENT 4:**

A prototype of protein chip technology compatible with SIMS has been developed to study the interaction of protein A (*S.aureus*) synthesized in a recombinant system *E.coli* and a commercial antibody.

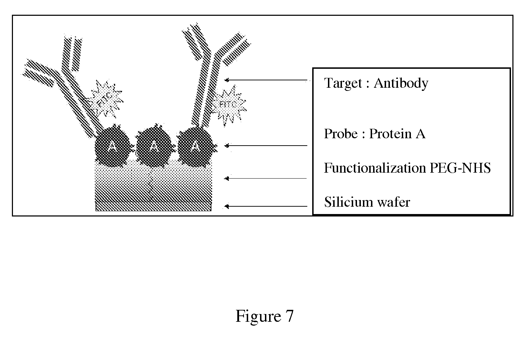

The support consists of a silicon wafer covered with a film of PEG functionalized with N-hydroxysuccinimide (NHS); the $^{15}N$-labeled protein A (probe) is thus covalently attached to PEG (see Figure 7). These probes are incubated with solutions of different concentrations of commercial antibodies. We can then establish a quantification of the signal depending on the amount of target that will interact with the labeled probes.

***Materials and methods***

*Productiort of protein A-enriched $^{15}N$*

A production strain *E.coli* BL21 (DE3) was transformed with the expression vector pGTPc500_6His8PSA (32-327) with selection on LB agar + kanamycin.

An isolated clone is then used to perform the production:

• Cultures are grown in 250mL Erlenmeyer flasks with 50 mL of culture medium M9. The only source of nitrogen of this medium is $^{15}N$ ammonium chloride. Agitation is 220rpm.

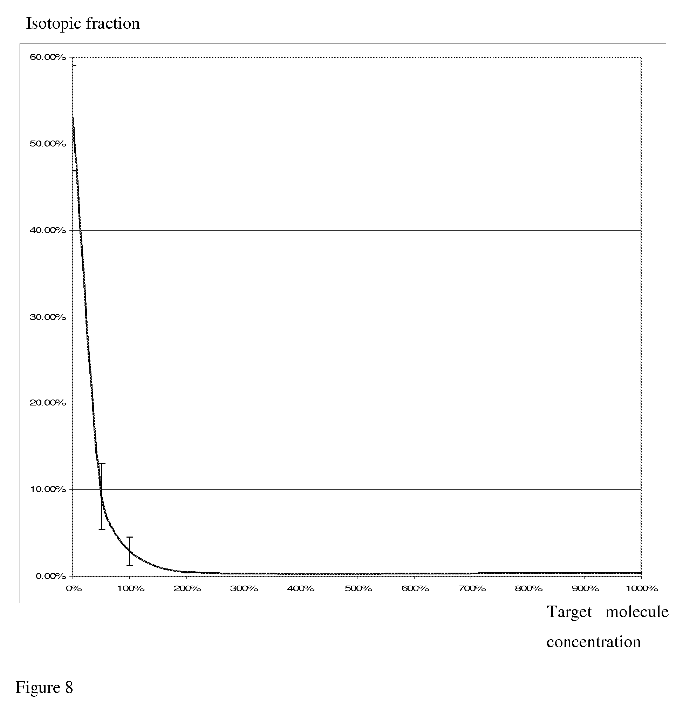

• Bacterial growth at 37°C is followed by measuring the absorbance at 600nm ($A_{600nm}$). The cultures were inoculated after overnight in selective medium M9 + kanamycin. The growth curve is shown in Figure 8.

• Induction is triggered by addition of IPTG (Isopropyl-1-thio-bD-galactopyranoside).

• The bacterial pellets were harvested by centrifugation 15 minutes at 14,000 rpm at 4°C.

• The bacterial pellets are taken up in 5 mL lysis buffer (PBS (50 mM Na*PO$_4$, 300 mM NaCl, pH 7.4) pH 7.4 and Triton X-100 0.2%).

• Lysozyme at a final concentration of 1mg/mL is added and the bacterial suspension was incubated for 40-45 minutes in ice.

• The suspension is sonicated (by sonotronde) in four cycles of 30 seconds (1 minute between each cycle) at a power of 50% for the first three cycles, then 80% for the last cycle.

• The soluble and insoluble fractions of the obtained lysate were separated by centrifugation 30 minutes at 18,000 rpm and at 4°C.

• The purification protocol used is as follows:

o Buffer A: 50mM Na*PO$_4$, 300 mM NaCl, pH 7.4, 40 mM Imidazole

o Buffer B: 50 mM Na*PO$_4$ 300 mM NaCl, pH 7.4, 500 mM Imidazole

o The sample to purify: supernatant with the addition of imidazole, qs 40 mM final.

o Column: "Chelating His-Trap" 1ml loaded with nickel.

| Step | Description | Volume VdC | Outflow (mL/min) | Fractions |
|---|---|---|---|---|
| Equilibration | Buffer A | 10 | 0,8 | / |
| Fixation | Sample | 4 ml | 0,8 | Not retained |
| Washing | Buffer A | 20 | 0,8 | 5 x 4 ml |
| Elution | Buffer B | 10 | 0,8 | 10 x 1 ml |

• SDS page gel analysis and Western blot.

*Covalent attachment of protein A on silicon wafer*

1μL of a solution of protein A at 0.33 μg/μL enriched in $^{15}$N (isotopic percentage of 88 ± 4%) is deposited on a silicon wafer functionalized with PEG-NHS (Microsurfaces Inc., Minneapolis, USA). After 1 hour at room temperature (25°C), the surface is cleaned 5 times for 5 minutes with a solution of PBS pH 7.4 Tween-20 (0.1% v/v), and then 30 minutes with a solution of PBS pH 8 Tween-20 (0.1% v/v) and finally for 5 minutes with water (milli-Q).

*Incubation with the target protein (antibody)*

To establish a dilution range of the target molecule (antibody), we decided to do a solution with 50%, 100%, 200% and 1000% of target molecules, for a constant amount (in number of molecules) of probes (protein A). 10 μL of a solution of PBS-Tween-20 pH 7.4 containing 16.5 μg/μL of antibody (Sigma, Aldrich, Rockford, USA) was diluted with PBS-Tween-20 pH 7.4. Each solution was deposited on a spot of protein A and incubated for 1 h at room temperature, the surface of the wafer being cleaned 4 times for 5 minutes with a solution of PBS pH 7.4 Tween-20 (0.1% v/v) and 2 times for 5 minutes with water (milli-Q). The wafers are then dried under vacuum and coated with gold for 60 seconds with a Cressington sputter coater 108 auto.

*SIMS analysis and calculation of the isotopic fraction*

The surface of the wafers is analyzed with the NanoSIMS 50 in isotope ratio mode (fields 200 x 200μm$^2$, diaphragm D2). An incision was made to determine the location of the spot of protein A, a dozen of analysis are performed on each spot. The scan includes 600 measures, and weights of 12C14N and 12C15N are studied in parallel. For obtaining the isotopic fraction, we use the following formula:

$$\text{Isotopic fraction} = (\textstyle\sum \text{n}^{12}\text{C}^{15}\text{N}) / (\textstyle\sum \text{n}^{12}\text{C}^{15}\text{N} + \textstyle\sum \text{n}^{12}\text{C}^{14}\text{N}).$$

**Results**

**Experimental data**

By performing the curve of the isotopic fraction as a function of the amount of target molecules, there is a quick decrease of the fraction 12C15N: it rises from 52.93% ± 6% to less than 10% (9.17% ± 3.8%) when protein A interacts with a quantity of antibodies equal to 50% of the quantity of protein A. For comparable amounts of targets and probes, the isotopic fraction is significantly higher than the natural fraction: 2.89% $\pm$ 1.6% against 0.366%, whereas for higher concentrations of the target molecule, 200% and 1000%, the signal is very close to the natural fraction, respectively 0.43% $\pm$ 0.03% and 0.4% $\pm$ 0.01%. This strong decrease of the isotopic fraction is explained by the fact that the target molecule, the antibody, is a molecule of a size considerably greater than the probe molecule, respectively 150KDa cons 33KDa. Whenever an interaction occurs, the nitrogen amount will be greatly changed from 425 atoms of nitrogen for protein A to about 2500 for the protein A/antibody complex.

This demonstrates that the sensitivity of SIMS NADs chips are highly dependent on analyzed molecules, and this sensitivity will be higher when the target biomolecule is substantial in size vis-à-vis the probe biomolecule.

**Claims**

1. An array comprising a planar substrate having a conducting surface and a number of discrete areas containing molecular probes being labeled with at least one rare stable isotope selected in the group of $^{15}N$, $^{33}S$, $^{34}S$ and $^{36}S$, or with at least one exogenous isotope selected in the group of $^{79}Br$ and $^{81}Br$, wherein the array or the discrete areas are coated with a hydrophilic polymer that does not contain the common secondary ions $^{14}N$, $^{32}S$, $^{80}Br$, respectively, corresponding to the rare or exogenous isotope used for labeling the molecular probes.

2. An array comprising a planar substrate having a conducting surface and a number of discrete areas containing molecular probes being labeled with at least one rare, stable, isotope selected in the group $^2H$, $^{13}C$, $^{15}N$, $^{17}O$, $^{18}O$, $^{33}S$, $^{34}S$ and $^{36}S$, or with at least one rare, unstable, isotope selected in the group $^3H$ and $^{14}C$ or with at least one exogenous isotope selected in the group $^{79}Br$ and $^{81}Br$, wherein the array or the discrete areas are coated with a hydrophilic polymer that does not contain the common secondary ions $^1H$, $^{12}C$, $^{14}N$, $^{16}O$, $^{32}S$, $^{80}Br$, respectively, corresponding to the rare or exogenous isotope used for labeling the molecular probes, but that contains at least one of said rare stable isotope or exogenous isotope used for labeling the molecular probes.

3. An array according to claim **1** or **2,** wherein said planar substrate having a conducting surface is a silicon wafer.

4. An array according to anyone of claims **1** to **3,** wherein the hydrophilic polymer is selected in the group comprising monosaccharide, disaccharide, polysaccharide, synthetic hydrophilic polymer and proteins.

5. An array according to claim **4,** wherein the synthetic hydrophilic polymer is polyethylene glycol.

6. An array according to anyone of claim **2** to **5,** wherein said molecular probes are labeled with at least one stable isotope selected in the group $^{15}N$ $^{33}S$, $^{34}S$ and $^{36}S$.

7. An array according to anyone of claims **1** to **6,** wherein said array is a microarray wherein each discrete area has one of the dimensions length, width or diameter being from 1 $\mu$m to 1000 $\mu$m.

8. An array according to claim **7,** wherein each discrete area is a microwell that comprises molecular probes being labeled with at least one rare stable isotope selected in the group of $^{15}N$, $^{33}S$, $^{34}S$ and $^{36}S$, or with at least one exogenous isotope selected in the group of $^{79}Br$ and $^{81}Br$.

9. An array according to anyone of claims **1** to **6,** wherein said array is a nanoarray wherein each discrete area has one of the dimensions length, width or diameter being from 1 nm to 1000 nm.

10. An array according to claim **9,** wherein each discrete area is a nanowell that comprises molecular probes being labeled with at least one rare stable isotope selected in the group of $^{15}N$, $^{33}S$, $^{34}S$ and $^{36}S$, or with at least one exogenous isotope selected in the group of $^{79}Br$ and $^{81}Br$.

11. An array according to claim **8,** wherein each microwell comprises a number of nanowells, and said nanowells comprise the molecular probes being labeled with at least one rare stable isotope selected in the group o$_f$ $^{15}N$, $^{33}S$, $^{34}S$ and $^{36}S$, or with at least one exogenous isotope selected in the group of $^{79}Br$ and $^{81}Br$.

12. An *in vitro* method for detecting and quantifying in at least one sample the presence or absence of at least one biomolecule, comprising:

(a) contacting said at least one sample with an array according to anyone of claims **1** to **11**,

(b) washing and drying the array,

(c) detecting and counting by SIMS the common secondary ions along with the corresponding rare secondary ions.

13. *The in vitro* method according to claim **12,** wherein each sample to be tested is contacted with one or more discrete area of the array.

14. *The in vitro* method according to anyone of claims **12** to **13** for determining a molecular atlas of the sample tested, wherein said molecular atlas is the determination of the transcriptome, proteome, lipidome, metabolome, glycome and/or interactome of said sample.

15. *The in vitro* method according to anyone of claims **12** to **13** for predicting a predisposition to a disease, or for diagnosing a disease in a subject in need thereof, or for monitoring the efficacy of a therapeutic agent administrated to a subject to treat a disease, or for screening therapeutic agents.


**Patentansprüche**

1. Array, umfassend ein ebenes Substrat mit einer leitenden Oberfläche und einer Anzahl an diskreten Bereichen, die molekulare Sonden enthalten, die mit mindestens einem seltenen stabilen Isotop ausgewählt aus der Gruppe $^{15}$N, $^{33}$S, $^{34}$S und $^{36}$S oder mit mindestens einem exogenen Isotop ausgewählt aus der Gruppe $^{79}$Br und $^{81}$Br markiert sind, wobei der Array oder die diskreten Bereiche mit einem hydrophilen Polymer beschichtet sind, das nicht die gewöhnlichen Sekundärionen $^{14}$N, $^{32}$S, beziehungsweise $^{80}$Br enthält, entsprechend dem seltenen oder exogenen Isotop, das zum Markieren der molekularen Sonden verwendet wird.

2. Array, umfassend ein ebenes Substrat mit einer leitenden Oberfläche und einer Anzahl an diskreten Bereichen, die molekulare Sonden enthalten, die mit mindestens einem seltenen stabilen Isotop ausgewählt aus der Gruppe $^{2}$H, $^{13}$C, $^{15}$N, $^{17}$O, $^{18}$O, $^{33}$S, $^{34}$S und $^{36}$S, oder mit mindestens einem seltenen instabilen Isotop ausgewählt aus der Gruppe $^{3}$H und $^{14}$C, oder mit mindestens einem exogenen Isotop ausgewählt aus der Gruppe $^{79}$Br und $^{81}$Br markiert sind, wobei der Array oder die diskreten Bereiche mit einem hydrophilen Polymer beschichtet sind, das nicht die gewöhnlichen Sekundärionen $^{1}$H, $^{12}$C, $^{14}$N, $^{16}$O,$^{32}$S, beziehungsweise $^{80}$Br enthält, entsprechend dem seltenen oder exogenen Isotop, das zum Markieren der molekularen Sonden verwendet wird, sondern das mindestens eines der seltenen stabilen Isotope oder exogenen Isotope enthält, die zum Markieren der molekularen Sonden verwendet werden.

3. Array nach Anspruch 1 oder 2, wobei das ebene Substrat mit einer leitenden Oberfläche ein Siliciumwafer ist.

4. Array nach einem beliebigen der Ansprüche 1 bis 3, wobei das hydrophile Polymer ausgewählt ist aus der Gruppe umfassend Monosaccharid, Disaccharid, Polysaccharid, synthetisches hydrophiles Polymer und Proteine.

5. Array nach Anspruch 4, wobei das synthetische hydrophile Polymer Polyethylenglykol ist.

6. Array nach einem beliebigen von Anspruch 2 bis 5, wobei die molekularen Sonden mit mindestens einem stabilen Isotop ausgewählt aus der Gruppe $^{15}$N, $^{33}$S, $^{34}$S und $^{36}$S markiert sind.

7. Array nach einem beliebigen der Ansprüche 1 bis 6, wobei der Array ein Mikroarray ist, wobei jeder diskrete Bereich eine der Dimensionen Länge, Breite oder Durchmesser aufweist, die von 1 $\mu$m bis 1000 $\mu$m beträgt.

8. Array nach Anspruch 7, wobei jeder diskrete Bereich eine Mikrovertiefung ist, die molekulare Sonden umfasst, die mit mindestens einem seltenen stabilen Isotop ausgewählt aus der Gruppe $^{15}$N, $^{33}$S, $^{34}$S und $^{36}$S, oder mit mindestens einem exogenen Isotop ausgewählt aus der Gruppe von $^{79}$Br und $^{81}$Br markiert sind.

9. Array nach einem beliebigen der Ansprüche 1 bis 6, wobei der Array ein Nanoarray ist, wobei jeder diskrete Bereich eine der Dimensionen Länge, Breite oder Durchmesser aufweist, die von 1 nm bis 1000 nm beträgt.

10. Array nach Anspruch 9, wobei jeder diskrete Bereich eine Nanovertiefung ist, die molekulare Sonden umfasst, die mit mindestens einem seltenen stabilen Isotop ausgewählt aus der Gruppe $^{15}$N, $^{33}$S, $^{34}$S und $^{36}$S, oder mit mindestens

einem exogenen Isotop ausgewählt aus der Gruppe von $^{79}$Br und $^{81}$Br markiert sind.

11. Array nach Anspruch 8, wobei jede Mikrovertiefung eine Anzahl an Nanovertiefungen umfasst, und die Nanovertiefungen die molekularen Sonden umfassen, die mit mindestens einem seltenen stabilen Isotop ausgewählt aus der Gruppe $^{15}$N, $^{33}$S, $^{34}$S und $^{36}$S, oder mit mindestens einem exogenen Isotop ausgewählt aus der Gruppe von $^{79}$Br und $^{81}$Br markiert sind.

12. *In vitro*-Verfahren zum Nachweisen und Quantifizieren in mindestens einer Probe der Gegenwart oder Abwesenheit von mindestens einem Biomolekül, umfassend:

(a) Inkontaktbringen der mindestens einen Probe mit einem Array gemäß einem beliebigen der Ansprüche 1 bis 11,
(b) Waschen und Trocknen des Arrays,
(c) Nachweisen und Zählen durch SIMS der gewöhnlichen Sekundärionen neben den entsprechenden seltenen Sekundärionen.

13. *In vitro*-Verfahren nach Anspruch 12, wobei jede zu testende Probe mit einem oder mehreren diskreten Bereichen des Arrays in Kontakt gebracht wird.

14. *In vitro*-Verfahren nach einem beliebigen der Ansprüche 12 bis 13 zum Bestimmen eines molekularen Atlasses der gestesteten Probe, wobei der molekulare Atlas die Bestimmung des Transkriptoms, Proteoms, Lipidoms, Metaboloms, Glykoms und/oder Interaktoms der Probe ist.

15. *In vitro*-Verfahren nach einem beliebigen der Ansprüche 12 bis 13 zum Vorhersagen der Veranlagung für eine Krankheit oder zum Diagnostizieren einer Krankheit in einem dies benötigenden Subjekt, oder zum Überwachen der Wirksamkeit eines therapeutischen Mittels, das einem Subjekt verabreicht wird, um eine Krankheit zu behandeln, oder zum Screenen von therapeutischen Mitteln.

**Revendications**

1. Réseau comprenant un substrat plan ayant une surface conductrice et un certain nombre de zones discrètes contenant des sondes moléculaires étant marquées avec au moins un isotope rare stable choisi dans le groupe de $^{15}$N, $^{33}$S, $^{34}$S et $^{36}$S, ou avec au moins un isotope exogène choisi dans le groupe de $^{79}$Br et $^{81}$Br, dans lequel le réseau ou les zones discrètes sont recouverts d'un polymère hydrophile qui ne contient pas les ions secondaires courants $^{14}$N, $^{32}$S, $^{80}$Br, respectivement, correspondant à l'isotope rare ou exogène utilisé pour marquer les sondes moléculaires.

2. Réseau comprenant un substrat plan ayant une surface conductrice et un certain nombre de zones discrètes contenant des sondes moléculaires étant marquées avec au moins un isotope rare, stable, choisi dans le groupe de $^{2}$H, $^{13}$C, $^{15}$N, $^{17}$O, $^{18}$O, $^{33}$S, $^{34}$S et $^{36}$S, ou avec au moins un isotope rare, instable, choisi dans le groupe de $^{3}$H et $^{14}$C, ou avec au moins un isotope exogène choisi dans le groupe de $^{79}$Br et $^{81}$Br, dans lequel le réseau ou les zones discrètes sont recouverts d'un polymère hydrophile qui ne contient pas les ions secondaires courants $^{1}$H, $^{12}$C, $^{14}$N, $^{16}$O, $^{32}$S, $^{80}$Br, respectivement, correspondant à l'isotope rare ou exogène utilisé pour marquer les sondes moléculaires, mais qui contient au moins un dudit isotope rare stable ou isotope exogène utilisé pour marquer les sondes moléculaires.

3. Réseau selon la revendication 1 ou 2, dans lequel ledit substrat plan ayant une surface conductrice est une plaquette de silicium.

4. Réseau selon l'une quelconque des revendications 1 à 3, dans lequel le polymère hydrophile est choisi dans le groupe constitué par un monosaccharide, un disaccharide, un polysaccharide, un polymère hydrophile synthétique et des protéines.

5. Réseau selon la revendication 4, dans lequel le polymère hydrophile synthétique est un polyéthylène glycol.

6. Réseau selon l'une quelconque des revendications 2 à 5, dans lequel lesdites sondes moléculaires sont marquées avec au moins un isotope stable choisi dans le groupe de $^{15}$N, $^{33}$S, $^{34}$S et $^{36}$S.

**7.** Réseau selon l'une quelconque des revendications 1 à 6, dans lequel ledit réseau est un microréseau dans lequel chaque zone discrète a l'une des dimensions longueur, largeur ou diamètre étant de 1 $\mu$m à 1 000 $\mu$m.

**8.** Réseau selon la revendication 7, dans lequel chaque zone discrète est un micropuits qui comprend des sondes moléculaires étant marquées avec au moins un isotope rare stable choisi dans le groupe de $^{15}$N, $^{33}$S, $^{34}$S et $^{36}$S, ou avec au moins un isotope exogène choisi dans le groupe de $^{79}$Br et $^{81}$Br.

**9.** Réseau selon l'une quelconque des revendications 1 à 6, dans lequel ledit réseau est un nanoréseau dans lequel chaque zone discrète a l'une des dimensions longueur, largeur ou diamètre étant de 1 nm à 1 000 nm.

**10.** Réseau selon la revendication 9, dans lequel chaque zone discrète est un nanopuits qui comprend des sondes moléculaires étant marquées avec au moins un isotope rare stable choisi dans le groupe de $^{15}$N, $^{33}$S, $^{34}$S et $^{36}$S, ou avec au moins un isotope exogène choisi dans le groupe de $^{79}$Br et $^{81}$Br.

**11.** Réseau selon la revendication 8, dans lequel chaque micropuits comprend un certain nombre de nanopuits, et lesdits nanopuits comprennent les sondes moléculaires étant marquées avec au moins un isotope rare stable choisi dans le groupe de $^{15}$N, $^{33}$S, $^{4}$S et $^{36}$S, ou avec au moins un isotope exogène choisi dans le groupe de $^{79}$Br et $^{81}$Br.

**12.** Procédé *in* vitro pour la détection et la quantification dans au moins un échantillon de la présence ou l'absence d'au moins une biomolécule, comprenant :

(a) la mise en contact dudit au moins un échantillon avec un réseau selon l'une quelconque des revendications 1 à 11,
(b) le lavage et le séchage du réseau,
(c) la détection et le comptage par SIMS des ions secondaires courants ainsi que des ions secondaires rares correspondants.

**13.** Procédé *in vitro* selon la revendication 12, dans lequel chaque échantillon à tester est mis en contact avec une ou plusieurs zones discrètes du réseau.

**14.** Procédé *in vitro* selon l'une quelconque des revendications 12 à 13 pour déterminer un atlas moléculaire de l'échantillon testé, dans lequel ledit atlas moléculaire est la détermination du transcriptome, du protéome, du lipidome, du métabolome, du glycome et/ou de l'interactome dudit échantillon.

**15.** Procédé *in vitro* selon l'une quelconque des revendications 12 à 13 pour la prédiction d'une prédisposition à une maladie, ou pour le diagnostic d'une maladie chez un sujet en ayant besoin, ou pour la surveillance de l'efficacité d'un agent thérapeutique administré à un sujet pour traiter une maladie, ou pour le criblage d'agents thérapeutiques.

Figure 1

Figure 2

Figure 3

Figure 4

Figure 5

Figure 6

Target : Antibody

Probe : Protein A

Functionalization PEG-NHS

Silicium wafer

Figure 7

Isotopic fraction

Target molecule concentration

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LECHENE.** *Journal of Biology,* 2006, vol. 5, 20
- **LECHENE et al.** *Science,* 2007, vol. 317, 1563
- **BRANDT et al.** *Nucleic Acids Research,* 2003, vol. 31, 19
- **BELU et al.** *Anal. Chem.,* 2001, vol. 73 (2), 143-150
- *Proteomics,* 2005, vol. 5, 416-419